# EUROPEAN PATENT APPLICATION

(11) **EP 2 641 570 A1**
(43) Date of publication of application: **25.09.2013**
(21) Application number: 13160592.5
(22) Date of filing: 22.03.2013
(51) Int. Cl.: A61F 2/24

(54) **Replacement heart valve**

(30) Priority: 22.03.2012 GB 201205071
(71) Applicant: Brecker, Stephen, London NW8 6BR (GB)
(72) Inventor: Brecker, Stephen, London NW8 6BR (GB)
(74) Representative: Jacob, Reuben Ellis

(57) **Abstract**

A replacement heart valve, including a securing device (2) and a non-return valve (7), the securing device including a resiliently deformable material adapted to adopt collapsed and non-collapsed configurations, the resiliently deformable material having a memory such that it returns to the non-collapsed configuration when not held under tension, whereby the valve is adapted to be secured within the valve annulus. The securing device comprises two lobs connectedby a waist. Also featured is a method for replacing a heart valve and a kit, which uses this replacement heart valve together with a synching device for reshaping the coronary sinus.

## Description

The present invention relates to a replacement heart valve, and in particular to a transcatheter heart valve.

The valves of the heart control the flow of blood within the heart. When functioning correctly, the valves act as one-way valves to control the flow of blood between the chambers of the heart and to allow blood to flow out of the heart. The four valves of the heart are the tricuspid valve; the pulmonic, or pulmonary valve; the mitral valve; and the aortic valve.

Traditionally, when incorrectly functioning, heart valves have been replaced with surgical techniques involving open heart surgery and cardiopulmonary bypass. There are several disadvantages to this type of procedure such as impaired kidney and lung function, long recovery times, and secondary infection. The major disadvantage however is that the patient has to be placed onto a heart and lung machine (cardiopulmonary bypass) and the heart stopped.

An example of the incorrect functioning of a heart valve is mitral valve regurgitation. The mitral valve of the heart controls the flow of blood from the left atrium to the left ventricle. It is naturally formed from an anterior leaflet, and a posterior leaflet and a fibrous ring around the opening known as the mitral valve annulus. Mitral valve regurgitation is a condition in which the leaflets of the mitral valve no longer close properly leading to blood flowing back through the valve. The most common causes of this condition are mitral valve prolapse and valve ring dilatation. Conditions such as mitral valve regurgitation have led to the development of a wide range of replacement valve techniques.

As described in US3365728, one of the leading early replacement mitral heart valves was the Starr-Edwards heart valve. The replacement valve was placed into heart using open heart surgery and sutured into place in the mitral valve annulus. This replacement mitral valve comprised a caged ball. When blood flows from the left atrium into the left ventricle the ball would be pushed away from an opening and against the cage such that blood is pushed through the opening and when the left ventricle contracts and the left atrium is being refilled with blood, the ball would be pushed into the opening to close the valve. Beyond the disadvantages to open heart surgery stated above, use of this replacement heart valve could cause blood clots and patients fitted with this valve were required to take anti-coagulants. This type of replacement heart valve is no longer widely used.

Other mechanical and biological valves have been developed for open heart surgical replacement and, more recently, artificial heart valves that can be delivered by transcatheter techniques have become available. Thus far, these have been for implantation in the aortic and pulmonary position. These types of heart valves in the aortic position can be deployed in a retrograde manner from aorta into the left ventricular outflow tract, or in an antegrade fashion through the left ventricular apex. In order to perform this procedure in the retrograde manner, a collapsible heart valve is placed into a catheter and the catheter inserted into the femoral artery in a patient's leg. The catheter is then passed through the vascular system and guided to the patient's heart. Once positioned at the valve to be replaced the valve is deployed and expanded. The current designs of transcatheter valves fall into two major divisions - balloon expandable or self-expanding.

Now, a few designs of transcatheter valves have been developed that can be delivered into the mitral position. In such a transcatheter procedure, the catheter can be passed into the right atrium via the femoral vein and then passed from the right atrium into the left atrium via a puncture made in the intra-atrial septum. An alternative approach to the mitral valve will be via the transapical (retrograde) route.

Other methods to reduce mitral regurgitation include coronary sinus reduction methods. One such invention is described in US7637946B2. The device comprises an elongated member with proximal and distal anchors to secure the member in the coronary sinus adjacent to the mitral valve. This device applies tension within the coronary sinus, thereby creating a reshaping of the coronary sinus and, in turn, the posterior aspect of the mitral annulus. While this device may reduce or eliminate mitral valve regurgitation in some patients, it cannot be used alone where there is severe deterioration of the anterior and posterior leaflets.

Yet another approach is mitral valve repair using a device known as a MitraClip^{™}, as described in Maisano et al Multimedia Manual of Cardiothoracic Surgery Volume 2010, issue 0316. This publication describes transcatheter valve repair using a MitraClip^{™} which bonds the anterior and posterior leaflets at the site of regurgitation. The MitraClip^{™} fastens the leaflets together preventing regurgitation through the mitral valve. One disadvantage of this technique is that severe flail segments of valve cannot be treated.

It is an object of the present invention to provide an improved replacement heart valve, and in particular a transcatheter mitral valve replacement which can be delivered preferentially, transseptally from the right atrium to the left atrium or via the left ventricular apex. The design embodies the most desirable features of the transcatheter valve which are ease of deployment, retrievability, repositionability, and stability in the delivered position.

According to a first aspect of the invention, there is provided a device for replacing a heart valve comprising valve securing means and a non-return valve, the valve securing means comprising a resiliently deformable material adapted to adopt collapsed and non-collapsed configurations, the resiliently deformable material being such that the device returns to a non-collapsed configuration when not held under tension, whereby the valve is adapted to be secured within the valve annulus. This provides a self-securing and self-centring artificial valve deliverable by minimally invasive techniques for returning normal function to the heart.

Preferably, the securing means comprises two lobes connected by a waist, wherein the waist includes a non-return valve. The two lobes can be adapted to fit either side of the natural valve with the waist positioned in the opening. This allows the replacement valve to be secured in the correct position in the valve annulus. Also, as the valve is located on the inner surface of the valve securing means it is protected and secured in the same position as the original mitral valve.

Preferably, each lobe has an opening. The opening in the two lobes allows blood to flow through the valve.

Preferably, in use, the two lobes of the securing means are proximal and distal to the valve annulus, the proximal lobe contacting the annulus and the native anterior and posterior leaflets on the atrial side of the heart and the distal lobe contacting the annulus and the native anterior and posterior leaflets on the ventricular side of the heart. The proximal lobe on the atrium facing side of the device allows the valve to be secure in its position and press down evenly on the tissue around the valve opening when blood is being pushed through the device. Conversely, the distal lobe on the underside of the device allows it to maintain its position in the valve annulus when the atrium is being refilled with blood and the ventricle is contracting so causing the valve to close. Therefore, in the context of this invention, the proximal end of the device is the end which is located on the atrial side of the heart and the distal end of the device is the end located on the ventricular side of the heart such that blood flows from the proximal end to the distal end of the device and through the non-return valve.

Preferably, the lobes substantially define a disk in the non-collapsed configuration. This shape allows the device to be secured in the valve annulus when it returns to the non-collapsed configuration after being confined in a catheter in its collapsed configuration.

Preferably, the deployed diameter of the waist is between 18mm and 29mm. The size of the device required can vary from person to person and specialist measurements in this range may be used in order to fit the patient.

Preferably, the distal lobe is greater than or equal to the diameter of the proximal lobe. This aids the securing of the device due to the forces applied to the valve by the refilling of the left atrium.

Preferably, the proximal lobe forms a continuous segment that contacts the valve annulus and the native anterior and posterior leaflets on the atrial side of the heart. Having the proximal lobe form a continuous segment around the valve allows it to maintain its position in the valve annulus and support the valve on the atrium facing side of the device.

Preferably, the distal lobe forms a continuous segment that contacts the annulus and the native anterior and posterior leaflets on the ventriclular side of the heart. Having the distal lobe form a continuous segment around the valve on the underside of the device allows it to maintain its position in the valve annulus.

Preferably, the distal lobe forms an interrupted segment that contacts the annulus and the native anterior and posterior leaflets on the ventriclular side of the heart. The protrusions on the distal side of the replacement valve are adapted to fit around the *chordae tendineae* (also known as heart strings), which connect the valve to the papillary muscles, and allows them to function normally in the presence of the replacement valve. The gaps between the protrusions allow for normal functioning of the patient's native sub-valve apparatus (i.e. *chordae tendinae* and papillary muscles) on the ventricular side of the device.

Preferably, the gap between protrusions is from 2mm to 12mm. Different size gaps would apply to different sizes of valve, depending upon the requirements of the patient. For example, a valve for a patient with a large annulus may require larger gaps between the protrusions than a patient with a small annulus

The device is adapted to be confined within a catheter when held in its collapsed configuration. This allows the device to be passed into the heart using a catheter negating the need for open heart surgery. Suitable catheters would be deflectable catheters. The distal tips of these devices are movable and can be manipulated by an operator. These types of catheters are known and have been used previously to deploy other devices.

Preferably, the non-return valve consists of two or more leaflets. The size and shape of the device arrangement can be altered to suit the requirements of the patient. This provides flexibility in catering to the requirements of individual patients.

Preferably, the non-return valve consists of three leaflets. A three leaflet non-return valve arrangement allows for more flexibility of the valve when it is being deployed from the catheter.

Preferably, the securing means is a mesh. A mesh structure allows the replacement valve to be collapsible while also being durable in the deployed confirmation.

Preferably, the mesh is made from Nitinol. Nitinol is light, strong and can withstand the forces within the heart while retaining its shape.

Preferably, the device further comprises a waistband of material on the outer surface of the waist. This material prevents blood from flowing through the mesh pores such that the blood can only move through the valve and prevents blood from flowing back through the mesh of the securing means after it has passed through the valve, ie this will mitigate any paraprosthetic leak.

Preferably, the material located on the outer surface of the waist comprises Gore-Tex^{®} and/or polyester and/or gelfoam. All of these materials are suitable to prevent leakage of blood cells through the mesh pores.

Preferably, at least one sheet of material is located within one or both lobes. Having a material within the structure of the mesh will minimise any paraprosthetic leak.

Preferably, the at least one sheet of material is located within one or both lobes is Gore-Tex^{®} and/or polyester and/or gelfoam. These materials are suitable to prevent leakage of blood cells through the mesh pores.

Preferably, the device further comprises at least one catheter connection means. The catheter connection means allows the device to be deployed from its collapsed state and manipulated into the correct position in the valve annulus. The catheter connections means can also allow the securing means to be repositioned and/or retried by a device connected to the connection means.

Preferably, the catheter connection means is located on the proximal lobe for embodiments designed for trans-septal delivery and on the distal lobe for embodiments designed for transapical delivery. The catheter connection means allows the securing means to be deployed, repositioned and/or redeployed by a device connected to the catheter connection means.

Preferably, the catheter connection means comprises at least a screw or clip mechanism. A screw or clip mechanism will allow the device to be released from the delivery system in a reliable and safe manner.

Preferably, the distal lobe preferably comprises at least one or more stabilising protrusions. Distal lobe stability can be enhanced by the addition of stabilising protrusions that embed into the myocardium.

More preferably, each stabilising protrusion comprises one or more hooks or barbs. The stabilising hooks, or barbs are formed from short extensions of the nitinol which anchor the distal lobe to the myocardium.

Preferably, the device can be adapted for use with a synching device. A synching device ensures that the junction between the valve annulus and the device is tight and mitigates any paraprosthetic leak. A synching device can be placed within the coronary sinus in order to close the annulus around the device. This device can be specifically used with a synching device placed within the coronary sinus in order to close the annulus around the valve in the waist. This device lends itself to enhancement by use with a synching device as the synching device tightens the annulus around the waist of the device between the two lobes. If, for example, after deployment there is a significant paravalvular leak, then a synching device can be placed within the coronary sinus to reduce or eliminate the leak by applying external compressive force to the waist of the transcatheter valve.

According to the second aspect of the invention there is provided a kit comprising the device and a synching device. As previously stated, This device lends itself to enhancement by use with a synching device.

The invention will further be described by way of example only, with reference to the following drawings, in which:
Figure 1 is a cross section of the invention in use;
Figure 2 is a longitudinal view of the invention constrained in a catheter prior to being deployed;
Figure 3 is a plan view of the invention, as it would appear in use;
Figure 4 is a cross section of the invention showing the waistband material sutured onto the outer surface of the waist;
Figure 5 is a plan view of the device in its non-collapsed configuration;
Figure 6 is a plan view of the invention in its non-collapsed configuration and has an interrupted distal lobe; and
Figure 7 is a plan view of the invention in its non-collapsed configuration, wherein the distal lobe is formed from an interrupted segment.

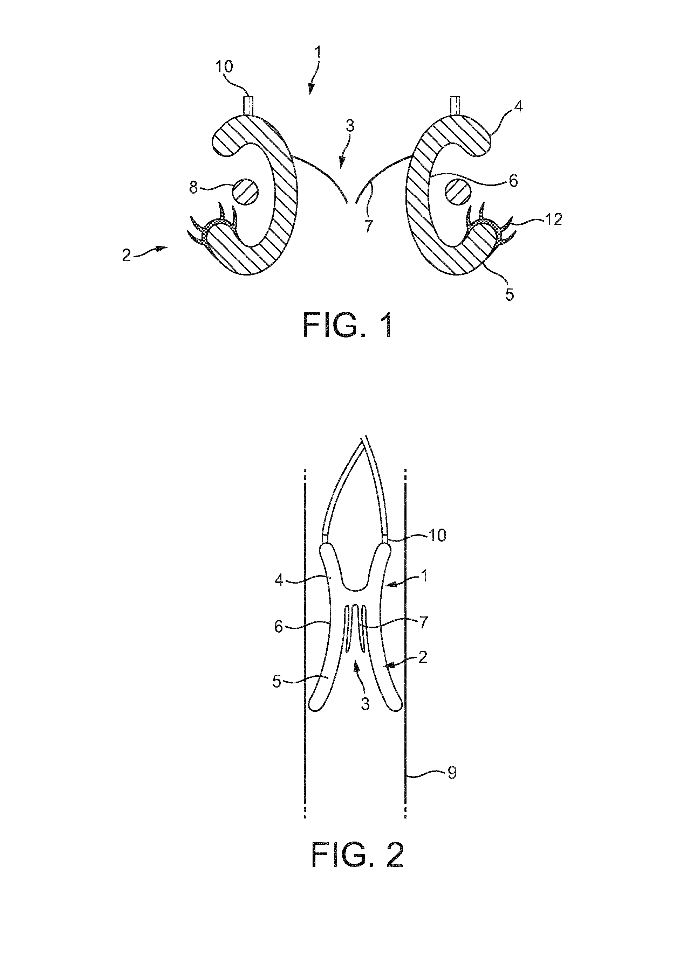

Figure 1 shows a device (1) for replacing a heart valve, in this example a mitral valve, comprising a securing means (2) and a non-return valve (3), the securing means (2) comprising a resiliently deformable material adapted to adopt collapsed and non-collapsed configurations, the resiliently deformable material having a memory such that it returns to a non-collapsed configuration when held under tension, wherein the valve is adapted to be secured within the mitral annulus (8).

The securing means (2) comprises a proximal lobe (4) and a distal lobe (5) with a waist portion (6) there between. Located within the waist portion (6) is a non-return valve (3). The non-return valve (3) comprising two or three leaflets (7) arranged such that fluid can pass through the valve in one direction but not pass in the opposing direction. The two or three leaflets of the valve allow the valve a high degree of flexibility and allows for the valve to be more easily retrieved. Located on the proximal lobe (4) are catheter connection means (10) which allow the device to be deployed from a catheter. Located on the distal lobe (5) are stabilising protrusions (12) which help to embed the device in myocardium.

Figure 2 shows the replacement mitral heart valve (1) constrained in a catheter (9) in a collapsed configuration. The replacement mitral valve (1) is restrained in a collapsed configuration by the walls of a catheter tube (9). The securing means (2) contains at least one catheter connection means (10) such that the securing means can be deployed, repositioned and/or retried by a device connected to the catheter connection means (10). The securing means (2) comprising a proximal lobe (4), a distal lobe (5) and a non-return valve (6), the non-return valve having two of more leaflets (7)

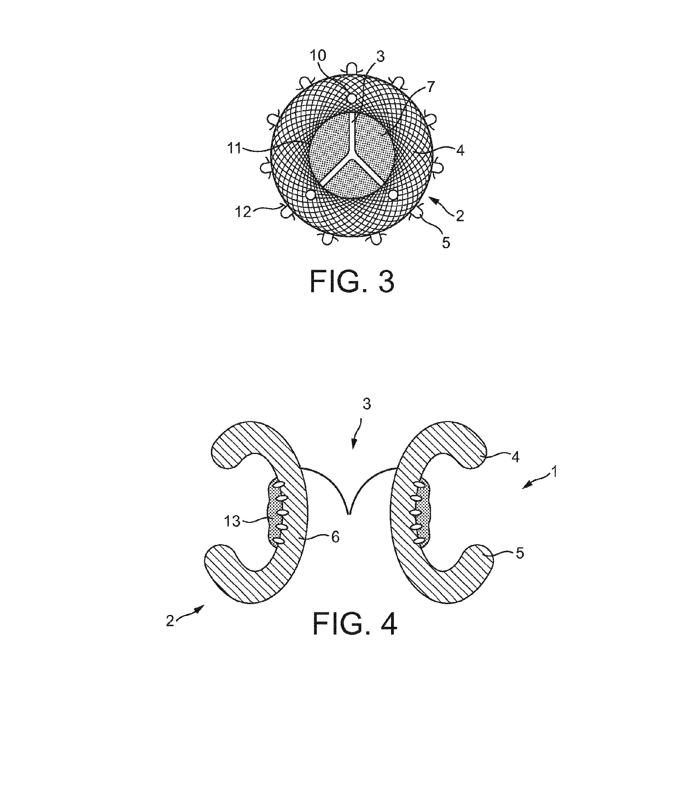

Figure 3 shows a plan view of one embodiment of the replacement mitral valve (1). The top of the replacement mitral valve has an opening (11) in the securing means (2). The securing means (2) is formed from a nitinol mesh and the top down view of this mesh of the proximal lobe (4) can be seen in the diagram. The stabilising protrusions (12) of the distal lobe (5) can be seen extending from below the circumference of the proximal lobe (4). Positioned at regular intervals around the circumference of the proximal lobe (4) are catheter connection means (10). Viewed through the opening in the securing means (11) the non-return valve (3) located in the waist (5) region can be seen. One possible arrangement of the non-return valve leaflets (7) is shown. In this embodiment there are three non-return valve leaflets (7) in a tricuspid arrangement.

Figure 4 shows the replacement mitral valve (1) comprising a valve securing means (2) and a non-return valve (3). The securing means (2) comprises a proximal lobe (4) and a distal lobe (5) with a waist portion (6) there between. The waist (6) having material (13) sutured to its outer surface. This material may be Gore-Tex^{®} and / or polyester, and / or Gelfoam.

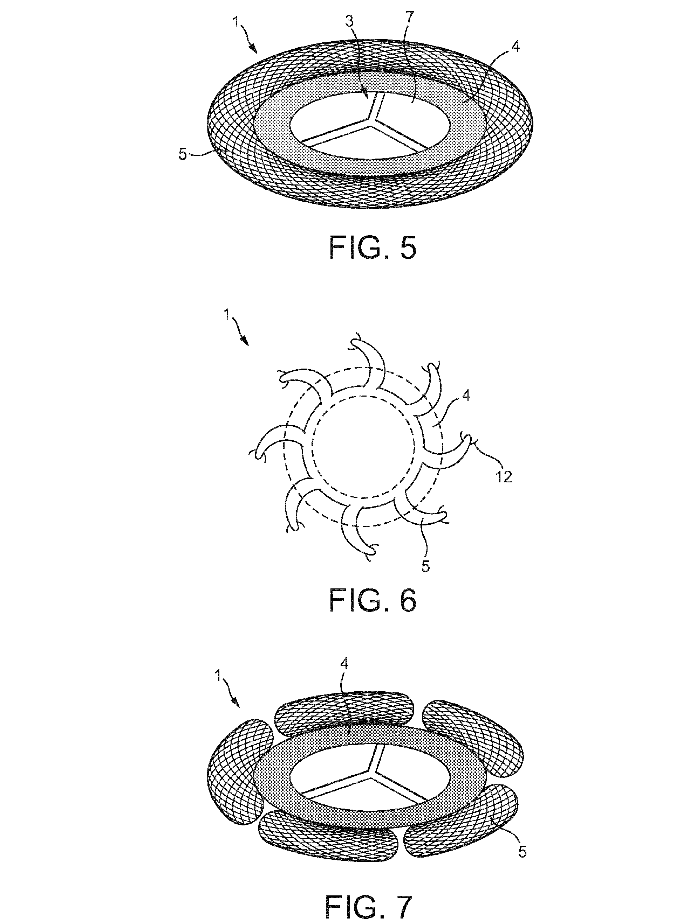

In the embodiment of Figure 5 the replacement mitral valve (1) is shown in its non-collapsed configuration. In this embodiment the distal lobe (5) is larger than the proximal lobe (6) and the three leaflets (7) of the valve (3) can be seen within the opening of the proximal lobe (4).

In the embodiment of Figure 6 the replacement mitral valve (1) is shown in its non-collapsed configuration and has an interrupted distal lobe (5)) Figure 6 is a plan view as if viewed through the proximal lobe (4). In the embodiment shown in this Figure the interrupted segments of the distal lobe (2) have a large gap between them. The distal lobe (5) can be seen and the stabilising protrusions (12) are shown extending outwardly from the distal lobe (5).

In the embodiment of Figure 7 the replacement mitral valve (1) is again shown in its non-collapsed configuration with an interrupted distal lobe (5) but the gaps between the segments is much smaller than the embodiment shown in Figure 6

In use, the replacement mitral heart valve is stretched to a collapsed configuration and restrained in that configuration within a catheter by using the catheter connection means. Once the replacement valve has been loaded into a catheter it can be inserted into the vascular system and passed to the heart. In the preferred transcatheter technique the catheter enters a vein, commonly the femoral vein, and is passed to the right atrium and then into the left atrium via a puncture made in the intra-atrial septum. As an alternative, the catheter could be delivered via the transapical route using transcatheter techniques.

Once in the left atrium, the catheter is positioned such the entrance of the catheter is above the mitral valve. After entering the left atrium with the delivery catheter, the entrance of the catheter is positioned at the mitral valve annulus and the device is pushed out of the entrance of the catheter using the catheter connection means. The connection means allow the replacement mitral valve to be manipulated. They can be used to push the restrained collapsed valve out of the catheters opening and then allow the device to be opened out into the mitral valve annulus. An important feature of this invention is that it is retrievable into the catheter such that the catheter can then be repositioned and the device redeployed.

As the device begins to be released from its restrained configuration, the device will begin to form its non- collapsed configuration. The securing means can self-centre the device in the opening by placing a compressive force on the annulus as the proximal lobe (3) and distal lobe (4) attempts to return to the uncollapsed state. The major mechanism of stabilising the device is the proximal and distal lobes, which are oversized in respect to the native mitral annulus. However, the waist of the device matches the native mitral annulus dimension. The lobes overhang the native annulus and the waist exerts a small degree of radial force by being slightly oversized with respect to the native annulus dimension. By this means and the natural stability afforded by the oversizing of the lobes the device will remain stable in position. In addition the distal lobe stability can be enhanced by the addition of stabilising hooks, or barbs - short extensions of the nitinol which embed into the myocardium.

Additionally, the waist (5) causes a radial force pushing outwards on the mitral valve annulus as the waist attempts to return to an uncollapsed state. These forces secure the replacement valve and ensure it is held in the centre of the opening. The device can be repositioned and/or redeployed by the catheter connection means and this allows the operator performing the procedure to ensure that the non- return valve is properly located in the mitral valve annulus. Therefore, the device can be deployed in a functional state immediately and no additional procedures, such as expanding the device using a balloon, are required following deployment from the catheter.

In certain embodiments there is a waistband of material on the outer surface of the waist. This waistband provides extra support and sealing to the tissues surrounding the mitral valve annulus.

Once the device has been properly positioned the catheter can be severed from the catheter connections means and the catheter withdrawn from the heart. The device will be fully deployed with blood flowing from the proximal end of the device to the distal end of the device and through the non-return valve in the waist. The device will remain in place and, due to the materials used, such as nitinol, be stable and durable.

This device can be specifically used with a synching device placed within the coronary sinus in order to close the mitral annulus around the non-return valve. The device of this invention lends itself to enhancement by use with a synching device. If, for example, after deployment there is a significant paravalvular leak, then a synching device can be placed within the coronary sinus to reduce or eliminate the leak by applying external compressive force to the waist of the transcatheter valve.

Although in the above example the invention has been described as a replacement mitral valve, it will be understood by the skilled worker that with suitable modification it can equally be a replacement tricuspid, pulmonary or aortic valve.

## Claims

1. A replacement heart valve, comprising securing means and a non-return valve, the securing means comprising a resiliently deformable material adapted to adopt collapsed and non-collapsed configurations, the resiliently deformable material having a memory such that it returns to the non-collapsed configuration when not held under tension, whereby the valve is adapted to be secured within the valve annulus.

2. The valve of claim 1, wherein the securing means comprises two lobes connected by a waist, wherein the waist includes the non-return valve and wherein each lobe has an opening.

3. The valve of any of the preceding claims, wherein, in use, the two lobes of the valve securing means are proximal and distal to the valve annulus, the proximal lobe contacting the valve annulus and the native anterior and posterior valve leaflets on the atrial side of the heart and the distal lobe contacting the valve annulus and the native anterior and posterior valve leaflets on the ventricular side of the heart.

4. The valve of claim 3, wherein the lobes substantially define a disk in the non-collapsed configuration.

5. The valve of claims 4, wherein the distal lobe is greater than or equal to the diameter of the proximal lobe.

6. The valve of claims 4 or 5, wherein the proximal lobe of the distal lobe forms a continuous segment that contacts the annulus and the native anterior and posterior leaflets on the atrial side of the heart.

7. The valve of claims 4, 5, or 6, wherein the distal lobe forms an interrupted segment that contacts the annulus and the native anterior and posterior leaflets on the ventricular side of the heart.

8. The valve of any of the preceding claims, wherein the valve can be confined within a catheter when held in its collapsed configuration.

9. The valve of any of the preceding claims, wherein the non-return valve consists of two or more leaflets.

10. The valve of any of the preceding claims, wherein the valve securing means comprises a mesh.

11. The valve of claims 2 to 10, further comprising a waistband of material on the outer surface of the waist.

12. The valve of the preceding claims, wherein at least one sheet of material is located within one or both lobes.

13. The valve of any of the preceding claims, further comprising at least one catheter connection means.

14. The valve of any of the preceding claims, wherein the distal lobe may comprise at least one or more stabilising protrusions.

15. The valve of any of the preceding claims, wherein the device is adapted for use with a synching device.

16. A kit comprising the valve of any preceding claim and a synching device.
